# EUROPEAN PATENT APPLICATION

(11) **EP 4 674 960 A1**
(43) Date of publication of application: **07.01.2026**
(21) Application number: 24763688.9
(22) Date of filing: 19.02.2024
(51) Int. Cl.: C12N 15/10, C12Q 1/686, G01K 5/48

(54) **STATUS IDENTIFICATION METHOD**

(30) Priority: 27.02.2023 JP 2023028534
(71) Applicant: Yokogawa Electric Corporation, Tokyo 180-8750 (JP)
(72) Inventor: MITSUMORI, Yuuji, Musashino-shi, Tokyo 180-8750 (JP); TAGUCHI, Tomoyuki, Musashino-shi, Tokyo 180-8750 (JP)
(74) Representative: Winter, Brandl - Partnerschaft mbB
(86) International application number: PCT/JP2024/005801
(87) International publication number: WO 2024/181207

(57) **Abstract**

In a state determination method, a bacterial culture solution S including bacteria C, and a foam bead B are enclosed in an enclosure container 20, the enclosure container 20 is sealed, heating is conducted, and an internal state related to at least one of a temperature and a pressure in the enclosure container 20 is determined based on a change in size of the foam bead B.

## Description

### Field

The present invention relates to a state determination method.

### Background

A technique (a high temperature and high pressure method, as appropriate), in which a bacterial culture solution is sampled, a solubilizing agent is added, and nucleic acid is extracted from cells of bacteria included in the bacterial culture solution, has been known. In another known technique, a temperature ("heating temperature" or "heating", as appropriate) in heating of a container in a process requiring heat treatment of the container is determined by a check of a color tone of a pigment, such as Prussian blue or a leuco dye, which changes in color according to temperature.

### Citation List

### Patent Literature

Patent Literature 1: Japanese Patent No. 5624487
Patent Literature 2: Japanese Patent Application Publication No. 2013-132298
Patent Literature 3: Japanese Patent Application Publication No. 2002-322385

### Summary

### Technical Problem

However, effectively determining an internal state, such as a heating temperature of a bacterial culture solution contained in a container is difficult with the above mentioned techniques. For example, in the above mentioned technique of checking a change in color of Prussian blue, the change in color occurs at a temperature around 121°C lower than a temperature around 140°C, at which nucleic acid is extractable from cells of bacteria, and checking whether a temperature around 140°C has been actually reached is thus difficult. Furthermore, in the above mentioned technique of checking a change in color of a leuco dye, the change in color occurs at around 70°C and the hue reversibly changes to the original color at 20°C, and checking whether a temperature around 140°C has been actually reached is thus difficult.

The present invention has been made in view of the above and an object thereof is to effectively determine a state of a sample.

### Solution to Problem

The present invention provides a state determination method of determining an internal state of a container, the state determination method including an enclosure process of enclosing a sample solution including a sample, and a bead formed by foaming, in the container, a heating process of sealing and heating the container, and a determination process of determining the internal state related to at least one of a temperature and a pressure in the container in the heating process based on a change in size of the bead.

### Advantageous Effects of Invention

The present invention has an effect of enabling an effective determination of a state of a sample.

### Brief Description of Drawings

FIG. 1 is a diagram illustrating an example of a configuration of a heating and pressurizing determination system according to an embodiment.
FIG. 2 is a diagram illustrating an example of experimental results of a heating and pressurizing checking experiment, according to the embodiment.
FIG. 3 is a diagram illustrating an example of a relation between temperature and pressure, according to the embodiment.
FIG. 4 is a diagram illustrating an example of first experimental conditions of a PCR amplicon measurement experiment, according to the embodiment.
FIG. 5 is a diagram illustrating an example of second experimental conditions of the PCR amplicon measurement experiment, according to the embodiment.
FIG. 6 is a diagram illustrating an example of third experimental conditions of the PCR amplicon measurement experiment, according to the embodiment.
FIG. 7 is a diagram illustrating an example of experimental results of the PCR amplicon measurement experiment, according to the embodiment.
FIG. 8 is a flowchart illustrating an example of a flow of a heating and pressurizing determination process according to the embodiment.

### Description of Embodiments

A state determination method according to an embodiment of the present invention will be described hereinafter in detail by reference to the drawings. The present invention is not to be limited by the embodiment described hereinafter.

### Embodiment

A configuration of a heating and pressurizing determination system 100, details of each process, and a flow of each process, according to an embodiment, will be described hereinafter in sequence and effects of the embodiment will be described lastly.

### 1. Configuration of Heating and Pressurizing Determination System 100

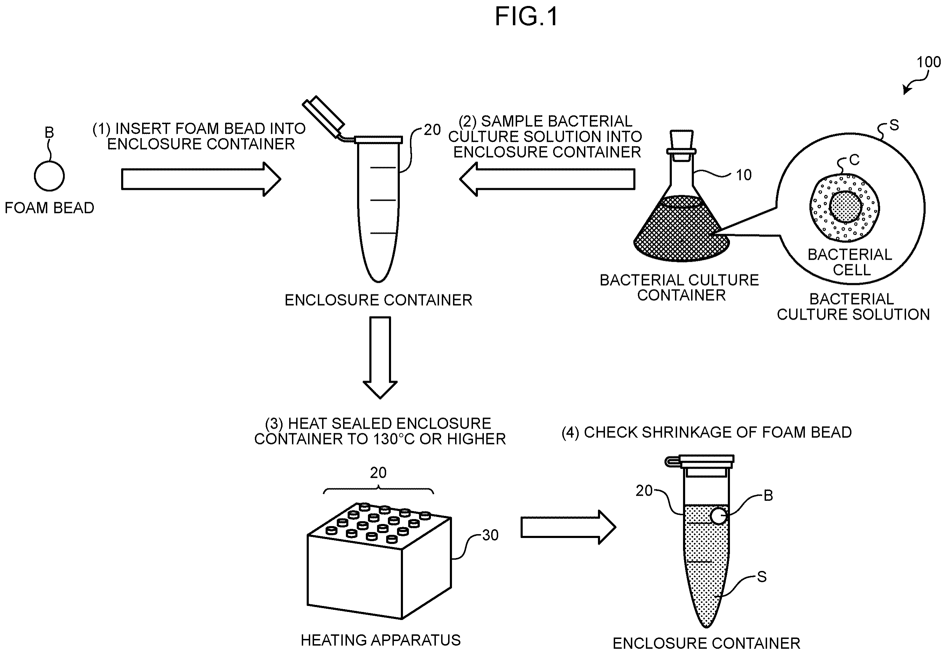

The configuration of the heating and pressurizing determination system 100 according to the embodiment will be described by use of FIG. 1. FIG. 1 is a diagram illustrating an example of the configuration of the heating and pressurizing determination system 100 according to the embodiment. An example of an overall configuration of the heating and pressurizing determination system 100, an example of processes in the heating and pressurizing determination system 100, and effects of the heating and pressurizing determination system 100 will be described hereinafter in this sequence.

### 1-1. Example of Overall Configuration of Heating and Pressurizing Determination System 100

The heating and pressurizing determination system 100 has a bacterial culture container 10, an enclosure container 20, and a heating apparatus 30. The bacterial culture container 10, the enclosure container 20, and the heating apparatus 30 will be described hereinafter in this sequence.

### 1-1-1. Bacterial Culture Container 10

The bacterial culture container 10 is a container to contain a bacterial culture solution S. The bacterial culture solution S is a solution where microbes, such as bacteria C, are cultured. In the example of FIG. 1, the bacterial culture container 10 is an Erlenmeyer flask with a stopper but the shape, material, and volume, for example, of the bacterial culture container 10 are not to be limited.

### 1-1-2. Enclosure Container 20

The enclosure container 20 is a container to enclose the bacterial culture solution S and a foam bead B. The foam bead B is a polystyrene-made bead formed by foaming. In the example of FIG. 1, the enclosure container 20 is a microtube but the shape, material, and volume, for example, of the enclosure container 20 are not to be limited.

### 1-1-3. Heating Apparatus 30

The heating apparatus 30 is a container to heat a heated solution H. In the example of FIG. 1, the heating apparatus 30 is a heating block, but the shape and material of and the heating method for the heating apparatus 30 are not to be limited.

### 1-1-4. Others

The heating and pressurizing determination system 100 illustrated in FIG. 1 may include a plurality of the bacterial culture containers 10, a plurality of the enclosure containers 20, or a plurality of the heating apparatuses 30. Furthermore, the bacterial culture container 10 may be configured to be integrated with the enclosure container 20.

### 1-2. Example of Processes in Heating and Pressurizing Determination System 100

The following description is on an example of processes in the heating and pressurizing determination system 100 described above. A foam bead insertion process, a bacterial culture solution sampling process, a heating process, and a size checking process will be described hereinafter in this sequence. These processes may be executed in a different sequence. Furthermore, some of these processes may be omitted.

### 1-2-1. Foam Bead Insertion Process

Firstly, the foam bead insertion process illustrated at (1) in FIG. 1 is implemented in the heating and pressurizing determination system 100. For example, in the foam bead insertion process, a foam bead B of expanded polystyrene is used and the foam bead B is inserted into an enclosure container 20. A plurality of the foam beads B may be inserted into the enclosure container 20 in this foam bead insertion process.

### 1-2-2. Bacterial Culture Solution Sampling Process

Secondly, the bacterial culture solution sampling process illustrated at (2) in FIG. 1 is implemented in the heating and pressurizing determination system 100. For example, in the bacterial culture solution sampling process, Escherichia coli (E. coli) or Staphylococcus aureus (S. aureus) is used as bacteria C, and part of a bacterial culture solution S that has been cultured overnight at 37°C in a soybean casein digest (SCD) liquid culture medium is sampled into the enclosure container 20 by use of a sterilized volumetric pipette. In this bacterial culture solution sampling process, a solubilizing agent that promotes dissolution of cells of the bacteria C may be added into the enclosure container 20 further.

### 1-2-3. Heating Process

Thirdly, the heating process illustrated at (3) in FIG. 1 is implemented in the heating and pressurizing determination system 100. For example, in the heating process, a heating apparatus 30 is preheated to a set temperature of 140°C, the enclosure container 20 is placed in the heating apparatus 30, and the enclosure container 20 having the foam bead B and the bacterial culture solution S enclosed therein is heated at 140°C for 45 seconds.

### 1-2-4. Size Checking Process

Fourthly, the size checking process illustrated at (4) in FIG. 1 is implemented in the heating and pressurizing determination system 100. For example, in the size checking process, the fact that the bacterial culture solution S in the enclosure container 20 has been heated to 130°C or higher is checked by a visual check of a reduction in size of the foam bead B inserted in the enclosure container 20. In this size checking process, from a relation between temperature and pressure estimated from a volume and contents of the enclosure container 20, a pressure applied to the interior of the enclosure container 20 may be determined.

### 1-2-5. Others

In the heating and pressurizing determination system 100, a nucleic acid purification process of purifying nucleic acid of the bacteria C extracted through the heating process may be implemented further. For example, in the nucleic acid purification process, the nucleic acid extracted from the bacteria C may be purified by injection of the bacterial culture solution S that has been subjected to the heating process into a column and injection of an eluate therein.

In the heating and pressurizing determination system 100, a nucleic acid amplification process of amplifying the nucleic acid of the bacteria C extracted through the heating process may be implemented further. For example, in the nucleic acid amplification process, the nucleic acid extracted from the bacteria C may be amplified by a PCR reaction through addition of a polymerase chain reaction (PCR) mix into the bacterial culture solution S that has been subjected to the heating process.

### 1-3. Effects of Heating and Pressurizing Determination System 100

Outlines of heating determination techniques that are reference techniques and points to be improved in the reference techniques will be described hereinafter in sequence and effects of the heating and pressurizing determination system 100 will be described thereafter.

### 1-3-1. Outline of First Reference Technique

In a first reference technique described in Patent Literature 2, a moist heating color change indicator composition is formed into ink and dates of manufacture and best before dates are printed on surfaces of packaging of retort pouch foods, the moist heating color change indicator composition including: (A) Prussian blue; (B) a gallate, such as propyl gallate; and (C) at least one kind of compound selected from a group consisting of: dicyandiamide; an amino acid, such as sodium glutamate; an aromatic carboxylic acid, such as benzoic acid; an acid amide, such as nicotinic acid amide; and a saccharide, such as starch. In the above mentioned first reference technique, the moist heating color change indicator composition has a blue color before heat sterilization treatment, and the heat sterilization treatment can be checked by the moist heating color change indicator composition having a black color after the treatment.

### 1-3-2. Points to be Improved in First Reference Technique

The following points are to be improved in the first reference technique. Firstly, in the first reference technique, a change in color at 121°C is checked. However, the treatment temperature effective in the nucleic acid extraction technique using the high temperature and high pressure method described in Patent Literature 1 is about 140°C and whether a temperature of about 140°C has been actually reached is thus unable to be checked with the first reference technique, in which the change in color occurs at 121°C. Secondly, in the first reference technique, a time period of about 20 minutes is needed for the pigment to change in color. However, the treatment time period for the nucleic acid extraction technique by the high temperature and high pressure treatment described in Patent Literature 1 is a few tens of seconds, and treatment longer than this will result in breakage of genomic DNA more than necessary and will highly likely influence the nucleic acid amplification process through the PCR reaction thereafter. In view of the above, applying the first reference technique as a temperature monitoring technique to the high temperature and high pressure method for extracting nucleic acid of bacteria C is difficult.

### 1-3-3. Outline of Second Reference Technique

According to a second reference technique described in Patent Literature 3, a thermosensitive hue-reversible composition that reversibly changes in hue by forming a color when heated and losing the color when cooled is provided, the thermosensitive hue-reversible composition containing three components that are: a color former including a reversible pigment that develops color in response to acid; a developer including a Lewis acid having a melting point in a temperature range of a desired hue change; and a sensitizer. In the second reference technique mentioned above, appropriate examples of the color former include a leuco dye, appropriate examples of the developer include a long chain carboxylic acid, and appropriate examples of the sensitizer include an acid amide.

### 1-3-4. Points to be Improved in Second Reference Technique

The following points are to be improved in the second reference technique. Firstly, in the second reference technique, the leuco dye changes in color at 70°C and the hue reversibly changes to the original color at 20°C. However, the treatment temperature effective in the nucleic acid extraction technique using the high temperature and high pressure method described in Patent Literature 1 is about 140°C and whether a temperature around 140°C has actually been reached is thus unable to be checked with the second reference technique, in which the color changes at 70°C. Secondly, because the leuco dye has a property of forming a color by reacting with an acid, the hue change may be influenced by properties of the reaction solution in the second reference technique and the leuco dye is thus difficult to be used as a process control. In view of the above, applying the second reference technique as a temperature monitoring technique to the high temperature and high pressure method for extracting nucleic acid of bacteria C is difficult.

### 1-3-5. Outline of Heating and Pressurizing Determination System 100

In the heating and pressurizing determination system 100, a bacterial culture solution S including bacteria C, and a foam bead B are enclosed in an enclosure container 20, heating is conducted after the enclosure container 20 has been sealed, and an internal state of the enclosure container 20 is determined on the basis of a change in size of the foam bead B, the internal state being related to at least one of a temperature and a pressure in the enclosure container 20. Furthermore, in the heating and pressurizing determination system 100, nucleic acid extracted from cells of the bacteria C by heating is purified. Furthermore, in the heating and pressurizing determination system 100, the nucleic acid extracted from the cells of the bacteria C by heating is amplified.

### 1-3-6. Effects of Heating and Pressurizing Determination System 100

Firstly, the heating and pressurizing determination system 100 enables a temperature state of a solution in a well-closed container to be checked thoroughly and directly. That is, because the foam bead B inserted in the enclosure container 20 significantly shrinks at 130°C or higher, the heating and pressurizing determination system 100 is expected to be applied as a temperature monitoring technique to an autoclave, a representative disinfection technique implemented at a temperature higher than 120°C.

Secondly, the heating and pressurizing determination system 100 facilitates checking of maximum temperatures reached. That is, colors of some of materials used as indicators return to their states before treatment when returned to room temperature after the treatment, but the change in the foam bead B at the maximum reached temperature equal to or higher than 130°C is able to be maintained and visually checked even if the foam bead B is returned to room temperature after the high temperature and high pressure treatment in the heating and pressurizing determination system 100, and applications as a convenient temperature monitoring technique and a process control can thus be expected.

Thirdly, replacement of the solvent after the extraction of the nucleic acid is not needed in the heating and pressurizing determination system 100. That is, in using the cellular contents after the extraction in a subsequent process, replacement of the solvent is not needed because there is no influence on the extract itself and the PCR enzyme in the heating and pressurizing determination system 100, and an application as a process control for the high temperature and high pressure method described above can thus be expected.

### 2. Details of Processes in Heating and Pressurizing Determination System 100

The following description is on details of processes in the heating and pressurizing determination system 100, the processes corresponding to the state determination method, which is illustrated in FIG. 1 and is for determining an internal state of a container. As to the processes according to the embodiment, a bacterial culture process, the foam bead insertion process, the bacterial culture solution sampling process, the heating process, the nucleic acid purification process, and the nucleic acid amplification process will be described hereinafter in this sequence.

### 2-1. Bacterial Culture Process

The bacterial culture process of culturing bacteria C serving as a sample will be described hereinafter, the bacterial culture process being a process to be implemented before the bacterial culture solution sampling process in the heating and pressurizing determination system 100.

### 2-1-1. Specific Example of Bacterial Culture Process

For example, in the bacterial culture process, the bacteria C are cultured in a bacterial culture solution S contained in a bacterial culture container 10. As to an example of a piece of equipment used in this culture, in the bacterial culture process, a sterilized glass-made Erlenmeyer flask with a stopper is used as the bacterial culture container 10 to culture the bacteria C. As to an example of the bacteria C to be cultured, in the bacterial culture process, Escherichia coli or Staphylococcus aureus is cultured. As to an example of conditions for the culture, in the bacterial culture process, the bacteria C are cultured overnight at 37°C in an SCD liquid culture medium.

### 2-1-2. Method of Culturing Bacterial Culture Solution S

The bacterial culture solution S used in the bacterial culture process described above is obtained by culture of a sample having nucleic acid. A method of culturing the sample is not particularly limited, and may be, for example, a method (solid phase culture), in which a filter that has collected the sample is directly placed on a solid culture medium and the sample is cultured via the filter. Furthermore, another method of culturing the sample may be, for example, a method (liquid phase culture), in which the sample is cultured in the presence of a solution having a liquid culture medium or a solid culture medium dissolved in water. Furthermore, the kind of the liquid culture medium or solid culture medium used may be selected according to the kind of the sample cultured and physiological conditions.

### 2-1-3. Sample of Bacterial Culture Solution S

The sample to be treated in the bacterial culture process described above is not particularly limited. For example, the sample to be treated may be microbes, cells of an animal that is not a microbe (for example, cells of an insect), plant cells, a mycoplasma, or a virus.

The microbes may be of, for example, at least one species selected from a group consisting of an Acinetobacter species, an Actinomyces species, an Aerococcus species, an Aeromonas species, an Alcaligenes species, a Bacillus species, a Bacteriodes species, a Bordetella species, a Branhamella species, a Brevibacterium species, a Campylobacter species, a Candida species, a Capnocytophagia species, a Chromobacterium species, a Clostridium species, a Corynebacterium species, a Cryptococcus species, a Deinococcus species, an Enterococcus species, an Erysipelothrix species, an Escherichia species, a Flavobacterium species, a Gemella species, a Haemophilus species, a Klebsiella species, a Lactobacillus species, a Lactococcus species, a Legionella species, a Leuconostoc species, a Listeria species, a Micrococcus species, a Mycobacterium species, a Neisseria species, a Cryptosporidium species, a Nocardia species, an Oerskovia species, a Paracoccus species, a Pediococcus species, a Peptostreptococcus species, a Propionibacterium species, a Proteus species, a Pseudomonas species, a Rahnella species, a Rhodococcus species, a Rhodospirillum species, a Staphylococcus species, a Streptomyces species, a Streptococcus species, a Vibrio species, an Yersinia species, a Methylobacterium species, a Ralstonia species, and a Sphingomonas species.

Some of the microbes mentioned above take the form of spores or sporules in some growth states. The form of the sample to be treated in the heating and pressurizing determination system 100 is not particularly limited. Furthermore, samples to be treated in the heating and pressurizing determination system 100 may be of one kind or two or more kinds.

### 2-2. Foam Bead Insertion Process

The foam bead insertion process, which is an enclosure process of enclosing a bead formed by foaming in an enclosure container 20, will be described hereinafter, the enclosure process being a process implemented before or after the bacterial culture solution sampling process in the heating and pressurizing determination system 100.

### 2-2-1. Specific Example of Foam Bead Insertion Process

For example, in the foam bead insertion process, a foam bead B of expanded polystyrene is enclosed in the enclosure container 20. In the foam bead insertion process, the foam bead B may be enclosed in the enclosure container 20 having the bacterial culture solution S sampled therein.

### 2-2-2. Foam Bead B

The material for the foam bead B is not particularly limited. That is, the material for the foam bead B may be any of foamed materials including, in addition to the above mentioned expanded polystyrene, expanded polyurethane, expanded polyethylene, and expanded polypropylene, for example, as long as the foam bead B is a foamed particle that shrinks at a predetermined temperature or higher.

Furthermore, the color of the foam bead B is not particularly limited. That is, the foam bead B may have any color that is able to be visually recognized, such as a red color, a blue color, a yellow color, or a white color.

Furthermore, the shape of the foam bead B is not particularly limited. That is, the foam bead B is preferably a sphere of about 5 mm but may have any size that enables the foam bead B to be inserted in the enclosure container 20, such as a PCR tube. Furthermore, the foam bead B may be any minute particle having a particle size in a certain range, may be an ellipsoid or a polyhedron, other than a sphere, may have an unlevel surface on the particle, or may have a hole penetrating the particle.

Furthermore, the foam bead B preferably does not produce any eluate that inhibits a biological reaction, such as a PCR reaction, when heated, but is not particularly limited.

### 2-3. Bacterial Culture Solution Sampling Process

The bacterial culture solution sampling process, in which the bacterial culture solution S, which is a sample solution, is enclosed, will be described hereinafter, the bacterial culture solution sampling process being a process that is implemented after the bacterial culture solution sampling process in the heating and pressurizing determination system 100.

### 2-3-1. Specific Example of Bacterial Culture Solution Sampling Process

For example, in the bacterial culture solution sampling process, part of the bacterial culture solution S contained in the bacterial culture container 10 is sampled into the enclosure container 20. As to an example of a piece of equipment to be used in this sampling, in the bacterial culture solution sampling process, the bacterial culture solution S is sampled by using a sterilized glass-made volumetric pipette as the piece of equipment for the sampling and using a sterilized glass tube with a stopper as the enclosure container 20.

### 2-3-2. Enclosure Container 20

The enclosure container 20 in the bacterial culture solution sampling process described above is not particularly limited. For example, the enclosure container 20 may be a plastic tube with a stopper, or a microtube, such as a PCR tube, other than a glass tube with a stopper. Furthermore, the enclosure container 20 may have any sealable structure having durability against temperatures up to about 180°C in the heating process described later.

### 2-3-3. Others

In the bacterial culture solution sampling process described above, the bacterial culture solution S may be a solution that has been pretreated before being sampled into the enclosure container 20. For example, the bacterial culture solution S may be a suspension including bacteria C that have been added with an enzyme and incubated for a certain time period. Furthermore, the bacterial culture solution S may be a suspension including bacteria C, which have been subjected to centrifugal separation by a centrifugal separator, and from which the culture medium component has been removed.

### 2-4. Heating Process

The heating process, which is implemented after the foam bead insertion process and bacterial culture solution sampling process in the heating and pressurizing determination system 100, will be described hereinafter, the heating process being a process, in which the enclosure container 20 is sealed and heated and nucleic acid is extracted from cells of the bacteria C serving as the sample.

### 2-4-1. Specific Example of Heating Process

For example, in the heating process, a solubilizing agent that promotes dissolution of cells is added to the bacteria C in the enclosure container 20 having the bacterial culture solution S and the foam bead B enclosed therein, the enclosure container 20 is sealed by closure of the top of the enclosure container 20, and nucleic acid is extracted from the cells of the bacteria C by heating of the sealed enclosure container 20 at 140°C for 45 seconds using the heating apparatus 30, such as a heat block.

### 2-4-2. Kinds of Solubilizing Agents

In the heating process in the heating and pressurizing determination system 100, the above described effects are achieved just by use of water, but for the purpose of more efficiently extracting the nucleic acid from the sample, at least one kind of solubilizing agent selected from a group consisting of a surfactant, an alkali, an acid, a redox agent, and a protein denaturant is preferably included, in addition to water. The solubilizing agent has the ability of causing dissolution of a membrane structure of the sample. The solubilizing agent acting on the membrane structure of the sample facilitates breakage of the sample and enables efficient extraction of the nucleic acid from the sample. The following description is on kinds of solubilizing agents.

### 2-4-2-1. Surfactant

A surfactant used as the solubilizing agent may be, for example, ionic or nonionic. A nonionic surfactant may be, for example, octylphenol ethoxylate (C14H22O(C2H4O)n). In the nucleic acid extraction process in the heating and pressurizing determination system 100, commercially sold octylphenol ethoxylate, which may be, for example, Triton X-100 (C14H22O(C2H4O)n, n = 100) manufactured by Sigma-Aldrich, may be used.

Furthermore, an ionic surfactant may be anionic, cationic, or zwitterionic. An anionic surfactant may be, for example, sodium dodecyl sulfate (SDS). A cationic surfactant may be, for example, cetyltrimethylammonium bromide (CTAB). A zwitterionic surfactant may be, for example, betaine. "Betaine" herein is a general term for any compound having: a positive charge and a negative charge at positions that are not adjacent to each other in the same molecule; an atom having the positive charge and having no dissociatable hydrogen atom bonded thereto; and no charge as the molecule as a whole. Representative examples of betaine include trimethylglycine.

### 2-4-2-2. Alkali

An alkali used as the solubilizing agent may be, for example, sodium hydroxide (NaOH) or potassium hydroxide (KHO).

### 2-4-2-3. Acid

An acid used as the solubilizing agent may be, for example, hydrochloric acid (HCl) or sulfuric acid (H2SO4).

### 2-4-2-4. Redox Agent

A redox agent used as the solubilizing agent may be, for example, a hydrogen peroxide solution, β-mercaptoethanol, or dithiothreitol.

### 2-4-2-5. Protein Denaturant

A protein denaturant used as the solubilizing agent may be, for example, guanidine hydrochloride or urea.

### 2-4-2-6. Others

A chelator may be used as a component of the solubilizing agent. A chelator used as the solubilizing agent may be, for example, ethylenediaminetetraacetic acid (EDTA).

Furthermore, among the solubilizing agents mentioned above, the solubilizing agent in the heating and pressurizing determination system 100 preferably includes a surfactant, and preferably includes any one or both of SDS and octylphenol ethoxylate.

For example, SDS may be used in a case where the nucleic acid extracted in the nucleic acid extraction process in the heating and pressurizing determination system 100 is desired to be detected highly sensitively. By contrast, in a case where the nucleic acid extracted in the nucleic acid extraction process in the heating and pressurizing determination system 100 is to be used in an enzymatic reaction inhibited by SDS, octylphenol ethoxylate that acts on the membrane structure of the sample more mildly than SDS may be used.

The solubilizing agent in the heating and pressurizing determination system 100 may include a buffer as needed. The buffer may be, for example, tris(hydroxymethyl)aminomethane hydrochloride (Tris-HCl).

### 2-4-3. Kinds of Nucleic Acids

The kinds of nucleic acids extracted in the heating process described above are not particularly limited. For example, a nucleic acid extracted may be a genome DNA that is a deoxyribonucleic acid (DNA), a messenger RNA that is a plasmid DNA or a ribonucleic acid (RNA), a transfer RNA, or a ribosomal RNA.

### 2-5. Size Checking Process

The size checking process implemented after the heating process in the heating determination system 100 will be described hereinafter, the size checking process being a determination process of determining an internal state on the basis of a change in size of the foam bead B, the internal state being related to at least one of a temperature and a pressure in the enclosure container 20 in the heating process.

### 2-5-1. Specific Example of Size Checking Process

For example, in the size checking process, from shrinkage of the foam bead B, it is determined that the temperature, at which the enclosure container 20 has been heated, is 130°C or higher. That is, in the size checking process, the fact that the bacterial culture solution S including the bacteria C has been heated to 130°C or higher is able to be confirmed in a case where the size of the foam bead B is found, by visual inspection, to have been significantly reduced in size and changed to a size that is half its original size or less. Because the size changes in response to a temperature of 130°C or higher, in the size checking process, the temperature, at which the bacterial culture solution S including the bacteria C has been heated, may be estimated, on the basis of the reduced size of the foam bead B.

Furthermore, in the size checking process, a pressure in the enclosure container 20 may be determined on the basis of a relation between temperature and pressure. That is, in the size checking process, it is able to be determined that a maximum pressure of 2757 hPa has been reached at a maximum temperature of 130°C, a maximum pressure of 3706 hPa at a maximum temperature of 140°C, a maximum pressure of 4906 hPa at a maximum temperature of 150°C, a maximum pressure of 6403 hPa at a maximum temperature of 160°C, a maximum pressure of 8249 hPa at a maximum temperature of 170°C, and a maximum pressure of 10498 hPa at a maximum temperature of 180°C.

### 2-6. Nucleic Acid Purification Process

The nucleic acid purification process implemented after the heating process in the heating determination system 100 will be described hereinafter, the nucleic acid purification process being a process, in which the nucleic acid extracted from the bacteria C is purified.

### 2-6-1. Specific Example of Nucleic Acid Purification Process

For example, in the nucleic acid purification process, the nucleic acid is purified by injection of an eluate for elution of the nucleic acid into a column having an adsorption carrier for adsorption of the nucleic acid after injection of the nucleic acid into the column.

### 2-7. Nucleic Acid Amplification Process

The nucleic acid amplification process implemented after the heating process or after the nucleic acid purification process, in the heating determination system 100, will be described hereinafter, the nucleic acid amplification process being a process, in which the nucleic acid extracted from the cells of the bacteria C is amplified.

### 2-7-1. Specific Example of Nucleic Acid Amplification Process

For example, in the nucleic acid amplification process, the nucleic acid extracted by the heating process is amplified by a PCR reaction of a solution including the nucleic acid. Furthermore, in the nucleic acid amplification process, the nucleic acid purified by the nucleic acid purification process is amplified by a PCR reaction of a solution including the nucleic acid.

### 3. Various Experimental Results

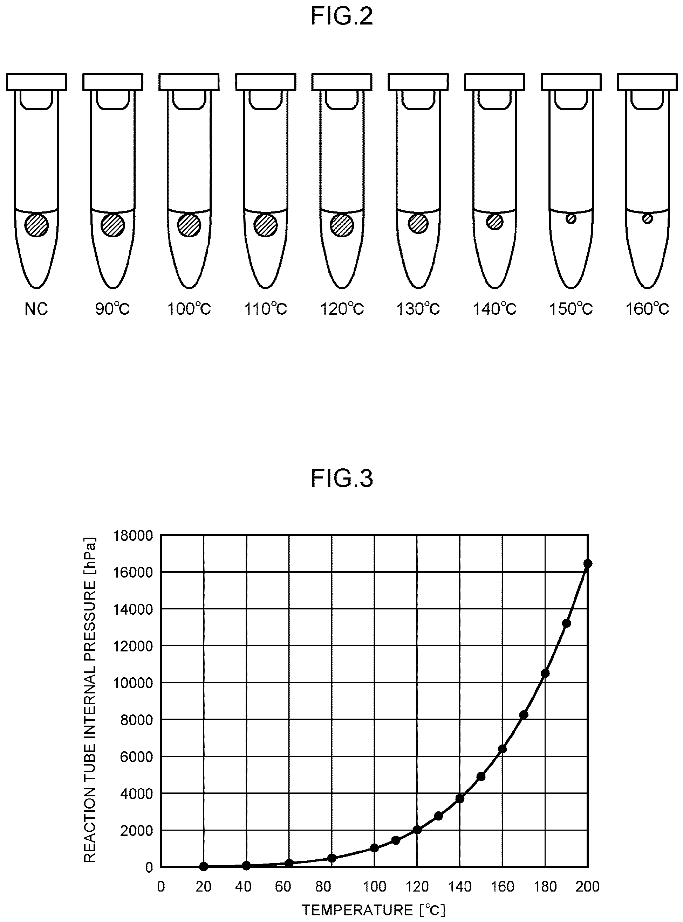

Results of various experiments using the heating and pressurizing determination system 100 according to the embodiment will be described by use of FIG. 2 to FIG. 7. Experimental results related to a heating and pressurizing checking experiment and experimental results related to a PCR amplicon measurement experiment will be described hereinafter in this sequence.

### 3-1. Heating and Pressurizing Checking Experiment

The experimental results related to the heating and pressurizing checking experiment for checking, for example, heating temperatures in the heating and pressurizing determination system 100 will be described by use of FIG. 2 and FIG. 3. FIG. 2 is a diagram illustrating an example of the experimental results of the heating and pressurizing checking experiment according to the embodiment. FIG. 3 is a diagram illustrating an example of a relation between temperature and pressure, according to the embodiment. An experimental procedure and experimental results of the heating and pressurizing checking experiment will be described hereinafter in sequence.

### 3-1-1. Experimental Procedure of Heating and Pressurizing Checking Experiment

The following description is on an example of an experimental procedure related to a heating checking experiment in the heating and pressurizing determination system 100. Firstly, a foam bead B, which is made of expanded polystyrene and is 6.5 mm, is inserted into an enclosure container 20, which is a PCR tube. Secondly, 40 µL of a solubilizing agent solution including 1% SDS and tris-HCl are injected into the PCR tube having the foam bead B inserted therein. Thirdly, the PCR tube enclosing the foam bead B and the solubilizing agent is sealed and heated for 60 seconds. Fourthly, a state of the foam bead B after the heat treatment is visually checked.

### 3-1-2. Experimental Results of Heating and Pressurizing Checking Experiment

The experimental results of the heating and pressurizing checking experiment will be described by use of FIG. 2. FIG. 2 illustrates states of PCR tubes after heating, for: a negative control sample, "NC", which is a mixed solution that has not been heated; "90°C", which is a mixed solution that has been heated at 90°C; "100°C", which is a mixed solution that has been heated at 100°C; "110°C", which is a mixed solution that has been heated at 110°C; "120°C", which is a mixed solution that has been heated at 120°C; "130°C", which is a mixed solution that has been heated at 130°C; "140°C", which is a mixed solution that has been heated at 140°C; "150°C", which is a mixed solution that has been heated at 150°C; and "160°C", which is a mixed solution that has been heated at 160°C.

### 3-1-3. Discussion on Experimental Results of Heating and Pressurizing Checking Experiment

In FIG. 2, a reduction in size of the foam bead B cannot observed for "NC, "90°C", "100°C", "110°C", and "120°C", but a significant reduction in size of the foam bead B can be visually recognized for "130°C", "140°C", "150°C", and "160°C". Furthermore, in FIG. 2, the sizes of the foam beads B have a relation, "130°C" > "140°C" > "150°C" > "160°C".

From the experimental results of the heating and pressurizing checking experiment, visually recognizing a change in size of a foam bead B in the heating and pressurizing determination system 100 enables a check of heating of each sample to a temperature of 130°C or higher.

### 3-1-4. Relation Between Temperature and Pressure

A relation between temperature and pressure inside the enclosure container 20 will be described by use of FIG. 3. As illustrated in FIG. 3, a theoretical relation between temperature (°C) and internal pressure in the reaction tube (hPa) can be derived from information on, for example, a volume of the enclosure container 20, a volume of the foam bead B, and components and amounts of its contents.

The example in FIG. 3 indicates that: the internal pressure in the reaction tube is "23 hPa" at the temperature of "20°C"; the internal pressure in the reaction tube is "73 hPa" at the temperature of "40°C"; the internal pressure in the reaction tube is "199 hPa" at the temperature of "60°C"; the internal pressure in the reaction tube is "475 hPa" at the temperature of "80°C"; the internal pressure in the reaction tube is "1022 hPa" at the temperature of "100°C"; the internal pressure in the reaction tube is "1450 hPa" at the temperature of "110°C"; the internal pressure in the reaction tube is "2018 hPa" at the temperature of "120°C"; the internal pressure in the reaction tube is "2757 hPa" at the temperature of "130°C"; the internal pressure in the reaction tube is "3706 hPa" at the temperature of "140°C"; the internal pressure in the reaction tube is "4906 hPa" at the temperature of "150°C"; the internal pressure in the reaction tube is "6403 hPa" at the temperature of "160°C"; the internal pressure in the reaction tube is "8249 hPa" at the temperature of "170°C"; the internal pressure in the reaction tube is "10498 hPa" at the temperature of "180°C"; the internal pressure in the reaction tube is "13210 hPa" at the temperature of "190°C"; and the internal pressure in the reaction tube is "16450 hPa" at the temperature of "200°C".

That is, a pressure applied to the interior of the enclosure container 20 is able to be determined from a heating temperature estimated from a size of the foam bead B after heating or a set temperature of the heating apparatus 30. In a case where a pressure applied to a foam bead B to be compared with has been given, a heating temperature may be determined from a size of the foam bead B that has been heated.

### 3-2. PCR Amplicon Measurement Experiment

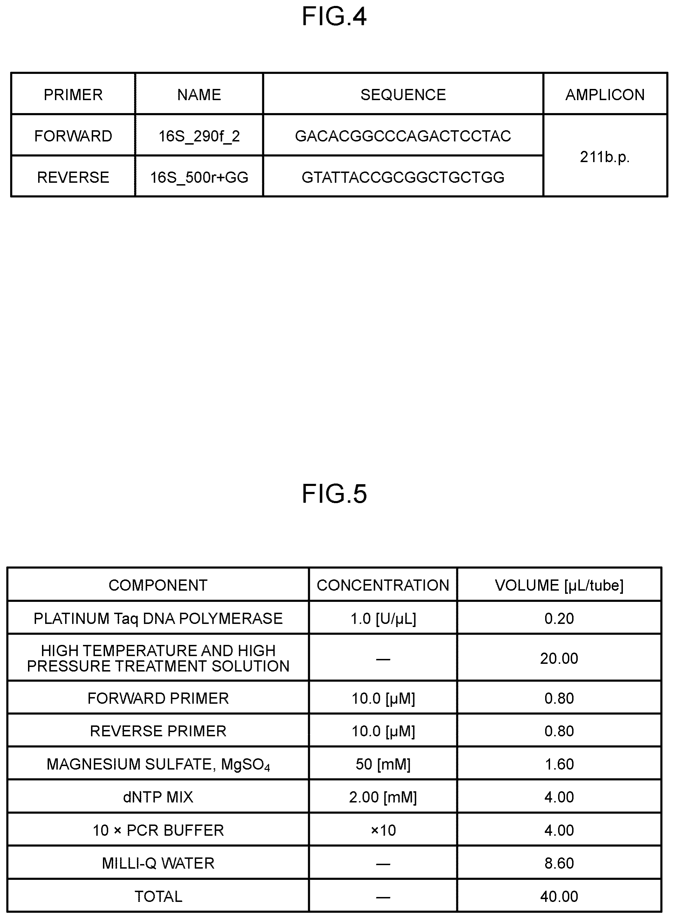

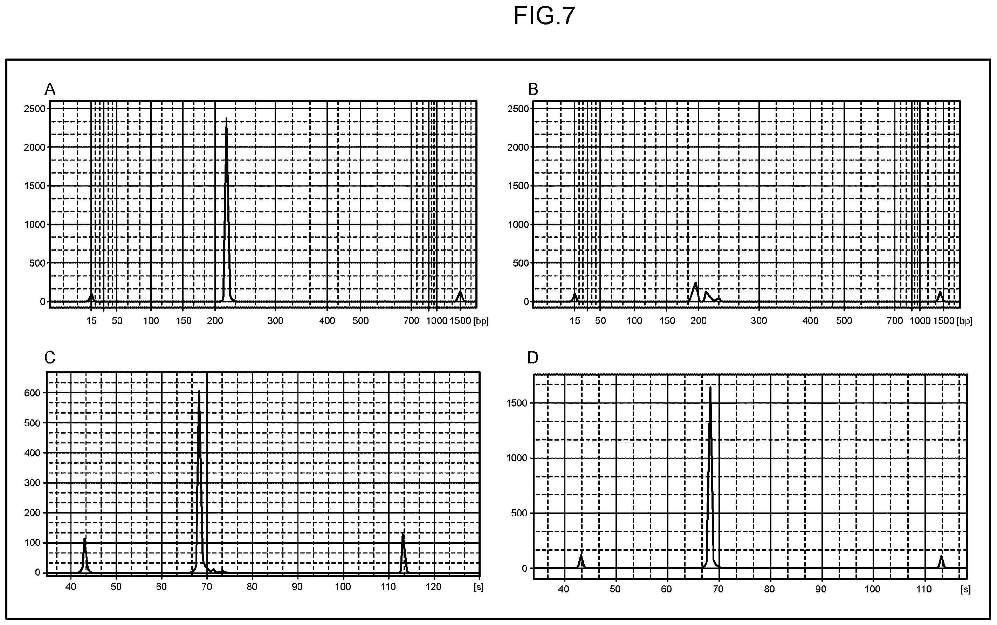

The experimental results related to the PCR amplicon measurement experiment for measuring PCR amplicons by means of the heating and pressurizing determination system 100 will be described by use of FIG. 4 to FIG. 7. FIG. 4 to FIG. 6 are diagrams illustrating examples of experimental conditions for the PCR amplicon measurement experiment according to the embodiment. FIG. 7 is a diagram illustrating an example of the experimental results of the PCR amplicon measurement experiment according to the embodiment. An experimental procedure and the experimental results of the PCR amplicon measurement experiment will be described hereinafter in sequence.

### 3-2-1. Experimental Procedure of PCR Amplicon Measurement Experiment

An example of the experimental procedure related to the PCR amplicon measurement experiment using the heating and pressurizing determination system 100 will be described by use of FIG. 4 to FIG. 6. Firstly, a foam bead B and a bacterial culture solution S are enclosed in an enclosure container 20 and the high temperature and high pressure treatment (heating at 140° for 45 seconds) by the high temperature and high pressure method is implemented. Secondly, a diluted solution is prepared by dilution of the bacterial culture solution S that has been subjected to the high temperature and high pressure treatment to 1/100. Thirdly, 20 µL of the diluted solution and 20 µL of a PCR mix described with respect to first or second experimental conditions is mixed together and a PCR reaction is conducted under temperature cycle conditions described with respect to third experimental conditions. Fourthly, electrophoresis of the solution after the PCR reaction is caused by use of "Agilent 2100 Bioanalyzer Electrophoresis System" manufactured by Agilent Technologies, Inc. and presence of PCR amplification is checked.

### 3-2-1-1. First Experimental Conditions

Firstly, the first experimental conditions, which are related to primers in the PCR amplicon measurement experiment, will be described by use of FIG. 4. As illustrated by the example in FIG. 4, a forward primer has a primer name, "16S 290f_2", and a base sequence, "GACACGGCCCAGACTCCTAC". Furthermore, a reverse primer has a primer name, "16S 500r+GG", and a base sequence, "GTATTACCGCGGCTGCTGG". Furthermore, the number of amplicon base pairs is "211b.p.".

### 3-2-1-2. Second Experimental Conditions

Secondly, the second experimental conditions, which are related to reagents in the PCR amplicon measurement experiment, will be described by use of FIG. 5. As illustrated by the example in FIG. 5, the reagents in the PCR amplicon measurement experiment, which are 0.20 µL/tube of 1.0 U/µL "Platinum Taq DNA Polymerase", 20 µL/tube of "high temperature and high pressure treatment solution", 0.8 µL/tube of 10.0 µM "forward primer", 0.8 µL/tube of 10.0 µM "reverse primer", 1.60 µL/tube of 50 mM "magnesium sulfate, MgSO4", 4.00 µL/tube of 2.00 mM "deoxynucleoside triphosphate (dNTP) mix", 4.00 µL/tube of "10 × PCR buffer" of a tenfold concentration, and 8.60 µL/tube of " Milli-Q Water", are mixed together to obtain a total of 40.00 µL/tube.

### 3-2-1-3. Third Experimental Conditions

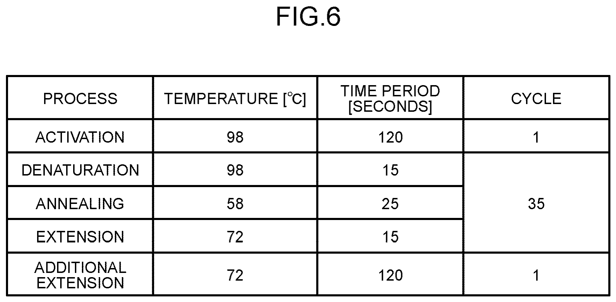

Thirdly, the third experimental conditions, which are related to reaction conditions of the PCR amplicon measurement experiment, will be described by use of FIG. 6. As to the reaction conditions of the PCR amplicon measurement experiment, as illustrated by the example in FIG. 6, one cycle of "activation" process is implemented under conditions, at 98°C for 120 seconds. A DNA elongation reaction includes three processes, which are "denaturation", "annealing", and "extension". The "denaturation" process is implemented under conditions, at 98°C for 15 seconds, the "annealing process" at 58°C for 25 seconds , and the "extension" process at 72°C for 15 seconds. These processes correspond to one cycle and 35 cycles are implemented. Furthermore, one cycle of an "additional extension" process is implemented under conditions, at 72°C for 120 seconds.

### 3-2-2. Experimental Results of PCR Amplicon Measurement Experiment

First experimental results of the PCR amplicon measurement experiment will be described by use of FIG. 7. FIG. 7A is an electrophoretogram of a positive control sample, "PC", which is a bacterial culture solution S with no foam bead B inserted therein. FIG. 7B is an electrophoretogram of a negative control sample, "PC", which is a bacterial culture solution S that has not been subjected to the high temperature and high pressure treatment. FIG. 7C is an electrophoretogram of "Foam Bead Method (with a solubilizing agent, 1/100 dilution)", which is a bacterial culture solution S having a foam bead B inserted therein and the solubilizing agent added therein. FIG. 7D is an electrophoretogram of "Foam Bead Method (with a solubilizing agent)", which is a bacterial culture solution S having a foam bead B inserted therein and the solubilizing agent not added therein.

### 3-2-3. Discussion on Experimental Results of PCR Amplicon Measurement Experiment

In FIG. 7, no significant reduction of PCR amplicons is observed in the result (FIG. 7D) of the PCR using the bacterial culture solution S having the foam bead B inserted therein in relation to the positive control sample, "PC", according to the result (FIG. 7A) of the PCR using the bacterial culture solution S having no foam bead B inserted therein.

From the experimental results of the PCR amplicon measurement experiment, it can be confirmed that there is no influence on the nucleic acid extraction process and the nucleic acid amplification process, in the heating and pressurizing determination system 100.

### 4. Flow of Processing in Heating and Pressurizing Determination System 100

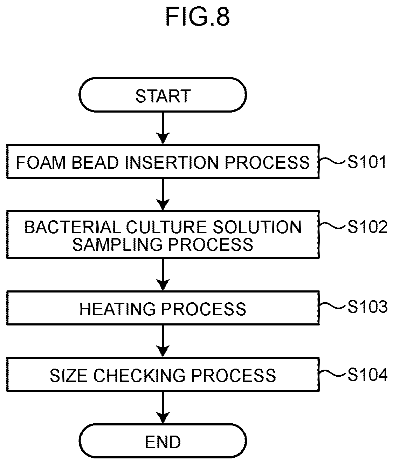

A flow of a process in the heating and pressurizing determination system 100 according to the embodiment will be described by use of FIG. 8. FIG. 8 is a flowchart illustrating an example of a flow of a heating and pressurizing determination process according to the embodiment. Steps S101 to S104 described below may be executed in a different sequence. Furthermore, some of Steps S101 to S104 described below may be omitted.

Firstly, in the heating and pressurizing determination system 100, the foam bead insertion process is implemented (Step S101). Secondly, in the heating and pressurizing determination system 100, the bacterial culture solution sampling process is implemented (Step S102). Thirdly, in the heating and pressurizing determination system 100, the heating process is implemented (Step S103). Fourthly, in the heating and pressurizing determination system 100, the size checking process is implemented (Step S104) and the heating and pressurizing determination process is ended. In the heating and pressurizing determination system 100, the nucleic acid purification process and the nucleic acid amplification process may be implemented after implementation of the size checking process.

### 5. Effects of Embodiment

Lastly, a description will be made on effects of the embodiment. First to seventh effects corresponding to the process according to the embodiment will be described hereinafter.

### 5-1. First Effect

Firstly, in the above described process according to the embodiment, a sample solution including a sample and a foam bead B are enclosed in an enclosure container 20, heating is conducted after the enclosure container 20 has been sealed, and an internal state related to at least one of a temperature and a pressure in the enclosure container 20 is determined on the basis of a change in size of the foam bead B. Therefore, the process according to the embodiment enables a state of the sample to be determined effectively.

### 5-2. Second Effect

Secondly, in the above described process according to the embodiment, from shrinkage of the foam bead B, it is determined that a temperature, at which the enclosure container 20 has been heated, is 130°C or higher. Therefore, the process according to the embodiment enables an effective determination of a state of the sample in heating treatment at 140°C or higher.

### 5-3. Third Effect

Thirdly, in the above described process according to the embodiment, the foam bead B is expanded polystyrene. Therefore, the process according to the embodiment enables a state of the sample to be determined effectively by use of the foam bead B made of a material that is readily available.

### 5-4. Fourth Effect

Fourthly, in the above described process according to the embodiment, the enclosure container 20 is a PCR tube. Therefore, the process according to the embodiment enables an effective determination of a state of the sample in treatment where a small amount of the sample is used.

### 5-5. Fifth Effect

Fifthly, in the above described process according to the embodiment, the sample solution is a bacterial culture solution S including bacteria C. Therefore, the process according to the embodiment enables an effective determination of a state of the sample in a high temperature and high pressure method for extracting nucleic acid from cells of the bacteria C.

### 5-6. Sixth Effect

Sixthly, in the above described process according to the embodiment, the nucleic acid extracted from the cells of the bacteria C is purified. Therefore, the process according to the embodiment enables an effective determination of a state of the sample without influencing the purification of the nucleic acid extracted from the cells of the bacteria C.

### 5-7. Seventh Effect

Seventhly, in the above described process according to the embodiment, the nucleic acid extracted from the cells of the bacteria C is amplified. Therefore, the process according to the embodiment enables an effective determination of a state of the sample without influencing the amplification of the nucleic acid extracted from the cells of the bacteria C.

### System

Any processing procedure, control procedure, specific name, and information including various data and parameters, which have been described above and illustrated in the drawings, may be optionally modified unless particularly stated otherwise.

Furthermore, the components of each device in the drawings have been illustrated functionally and/or conceptually, and are not necessarily physically configured as illustrated in the drawings. That is, specific modes of separation and integration of each device are not limited to those illustrated in the drawings. That is, all or part of each device may be configured to be functionally or physically separated or integrated in any units according to various loads and use situations.

### Reference Signs List

- 10: BACTERIAL CULTURE CONTAINER
- 20: ENCLOSURE CONTAINER
- 30: HEATING APPARATUS
- 100: HEATING AND PRESSURIZING DETERMINATION SYSTEM

## Claims

1. A state determination method of determining an internal state of a container, the state determination method comprising:
an enclosure process of enclosing a sample solution including a sample, and a bead formed by foaming, in the container;
a heating process of sealing and heating the container; and
a determination process of determining the internal state related to at least one of a temperature and a pressure in the container in the heating process based on a change in size of the bead.

2. The state determination method according to claim 1, wherein in the determination process, the temperature being 130°C or higher is determined from shrinkage of the bead, the temperature being a temperature that the container has been heated at.

3. The state determination method according to claim 1, wherein the bead is expanded polystyrene.

4. The state determination method according to claim 1, wherein the container is a polymerase chain reaction (PCR) tube.

5. The state determination method according to any one of claims 1 to 4, wherein the sample solution is a bacterial culture solution including a bacterium.

6. The state determination method according to claim 5, further including a purification process of purifying a nucleic acid extracted from cells of the bacterium through the heating process.

7. The state determination method according to claim 5, further including an amplification process of amplifying a nucleic acid extracted from cells of the bacterium through the heating process.
